(19) Europäisches Patentamt
European Patent Office
Office européen des brevets

(11) **EP 4 450 967 A1**

(12) **EUROPEAN PATENT APPLICATION**
published in accordance with Art. 153(4) EPC

(43) Date of publication:
**23.10.2024 Bulletin 2024/43**

(21) Application number: **22911192.7**

(22) Date of filing: **19.12.2022**

(51) International Patent Classification (IPC):
**G01N 31/00** (2006.01)   **G01N 31/12** (2006.01)
**G01N 33/2022** (2019.01)   **G01N 21/3563** (2014.01)

(52) Cooperative Patent Classification (CPC):
**G01N 21/3563; G01N 31/00; G01N 31/12;
G01N 33/2022**

(86) International application number:
**PCT/JP2022/046727**

(87) International publication number:
**WO 2023/120490 (29.06.2023 Gazette 2023/26)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **21.12.2021 JP 2021207422**

(71) Applicant: **JFE Steel Corporation
Tokyo 100-0011 (JP)**

(72) Inventor: **SUGAWARA, Seiya
Tokyo 100-0011 (JP)**

(74) Representative: **Hoffmann Eitle
Patent- und Rechtsanwälte PartmbB
Arabellastraße 30
81925 München (DE)**

(54) **METHOD FOR QUANTIFYING CARBON IN CARBIDE**

(57)    A method for quantifying carbon in carbides that allows highly precise and simple quantification of carbon in carbides. The method for quantifying carbon in the carbides includes a combustion process in which the carbides are heated together with a composite member in the presence of oxygen to oxidize the carbon in the carbides to carbon monoxide and/or carbon dioxide, and a quantification process of quantifying the carbon monoxide and/or the carbon dioxide. The composite member includes two or more metal members that have main components that are different from each other.

FIG. 1A

FIG. 1B

EP 4 450 967 A1

**Description**

TECHNICAL FIELD

The present disclosure relates to a method for quantifying carbon in carbides.

BACKGROUND

Amount and morphology of carbides in steel may significantly affect properties of steel. Therefore, by analyzing an amount of carbides, quantitatively evaluating carbide effects on a property of steel and controlling the property of the steel becomes possible. Conventionally, carbides have been quantified by quantifying metal elements bonded to carbon. However, the metal elements may exist as compounds such as nitrides, oxides, or sulfides in addition to carbides. In such cases, analysis of nitrogen, sulfur, and the like is also required to quantify carbides, resulting in a huge number of analytical procedures. Further, when quantifying a metal element and determining an amount of carbides from the quantitative results, the carbides are assumed to have a stoichiometric composition. In actual steel, however, carbides often do not take a stoichiometric composition, so an exact amount of carbides cannot be determined. Therefore, attempts are being made to directly quantify carbon in carbides in steel.

One method for quantifying carbon in steel is an infrared absorption method after combustion (based on Japanese Industrial Standard JIS G 1211-3, Part 3). Another method for extracting carbides from steel is electrolytic extraction. In electrolytic extraction, carbides are electrolytically extracted from steel, the carbides are then sieved into the solvent by ultrasound or the like to form a dispersion liquid, the dispersion liquid is then filtered to collect the carbides, and carbon in the collected carbides are quantified by an infrared absorption method after combustion.

However, contaminant carbon attaches to carbides collected by electrolytic extraction. There are three main types of contaminant carbon. The first is organic solvent carbon remaining in electrolytically extracted carbides, as the electrolyte and solvent used for electrolytic extraction of carbides are often organic solvents. The second is carbon produced by combustion of filters, as collection of carbides after electrolytic extraction is typically done by filtration using filters made of polycarbonate or other organic materials, and the filters are combusted along with the carbides. The third is carbon that attaches from the atmosphere. Such contaminant carbon is a source of error in quantifying carbon in carbides.

As a method for reducing this source of error, Patent Literature (PTL) 1 describes a method for analyzing carbides in steel in which, in quantitative analysis of carbides in a steel sample from electrolytic extraction residue, the electrolytic extraction residue is heated in a hydrogen atmosphere with a heating medium having high energy density in order to remove carbon content caused by organic matter present in the electrolyte, and then carbon in the electrolytic extraction residue is quantified.

PTL 2 describes a method for removing electrolyte, that is, contaminant carbon, from extraction residue of steel to be analyzed in quantitative analysis of carbon in carbides contained in the steel. According to the method, methyl alcohol is used for washing, followed by washing with warm water and organic solvent, and then heating and drying in air is performed. Further, PTL 2 describes a method of using an inorganic filter that has undergone a preheating treatment to remove contaminant carbon when collecting the extraction residue of the steel to be analyzed with a filter.

CITATION LIST

Patent Literature

PTL 1: JP H11-44687 A
PTL 2: JP 2004-257920 A

SUMMARY

(Technical Problem)

The method described in PTL 1 has a problem with precision as a method for quantifying carbon in carbides, because unstable carbides may be removed as well as contaminant carbon when removing carbon content caused by organic matter present in electrolyte.

The method described in PTL 2 is not only complicated because of the many washing processes, but also a removal rate of contaminant carbon may vary depending on the operator, and therefore precision as a method for quantifying carbon in carbides is problematic. Further, when carbides are collected using an inorganic filter that has undergone a preheating treatment to remove contaminant carbon and carbon in the carbides is quantified, carbides less than the pore size of the filter are not collected, meaning that quantifying carbon in fine carbides is not possible.

In view of the circumstances, it would be helpful to provide a method for quantifying carbon in carbides that allows highly precise and simple quantification of carbon in carbides.

(Solution to Problem)

The inventor conducted extensive studies on solutions to the problems described above.

Typically, when carbon is quantified by an infrared absorption method after combustion, a peak derived from contaminant carbon and a peak derived from carbon in carbides overlap, and therefore quantifying the carbon in the carbides is not possible. Therefore, the inventor first investigated a method to separate a peak derived from contaminant carbon from a peak derived from carbon in carbides when using an infrared absorption method after combustion. As a result, the inventor found that when a metal containing carbon is combusted, contaminant carbon is first oxidized to carbon monoxide and/or carbon dioxide and a peak derived from the contaminant carbon is detected, and then the carbon in the metal is oxidized to carbon monoxide and/or carbon dioxide and a peak derived from carbon in metal is detected. This is presumably because heat from heating is consumed by melting of the metal before the oxidation of the carbon in the metal, making it more difficult for the oxidation reaction of the carbon in the metal to occur. Based on this finding, the inventor attempted to combust carbides together with a metal member made of metal. As a result, the inventor found that it is possible to separate a peak derived from contaminant carbon from a peak derived from carbon in carbides by burning the carbides together with the metal member. Further, in a spectrum after appearance of a peak derived from contaminant carbon, only intensity due to background exists, and this intensity is extremely small and has little fluctuation, making it possible to quantify even trace amounts of carbon. However, it was found that the above effect is obtained when carbides is combusted together with a composite member and not when carbides is combusted together with a single metal member. Here, the composite member includes two or more metal members that have main components that are different from each other.

According to the method for quantifying carbon in carbides described above, a process of removing contaminant carbon from the carbides is not necessary, thus eliminating the need for complicated washing work that was conventionally required and providing operator-independent analysis values. Further, the method is applicable to carbon in carbides obtained by a method of extracting the carbides into a dispersion liquid and then extracting with a filter. Further, it was found that even fine carbides that cannot be collected by a filter may be collected by dropping the dispersion liquid onto a metal member and drying, and that carbon in the carbides may be quantified with high precision by combusting the metal member and the carbides. Accordingly, the inventor arrived at the present disclosure.

In order to solve the technical problems described above, the following are provided:

[1] A method for quantifying carbon in carbides, comprising: a combustion process in which the carbides are heated together with a composite member in the presence of oxygen to oxidize the carbon in the carbides to carbon monoxide and/or carbon dioxide; and a quantification process of quantifying the carbon monoxide and/or the carbon dioxide, wherein
the composite member includes two or more metal members that have main components that are different from each other.
[2] The method for quantifying carbon in carbides according to [1] above, wherein carbon content of each of the two or more metal members is 200 ppm or less.
[3] The method for quantifying carbon in carbides according to [1] or [2] above, wherein each of the two or more metal members is a sheet material having a thickness of 0.10 mm or less.
[4] The method for quantifying carbon in carbides according to [3] above, wherein the carbides are heated while wrapped in the sheet material in the combustion process.
[5] The method for quantifying carbon in carbides according to any one of [1] to [4] above, wherein an analysis method in the quantification process is an infrared absorption method.
[6] The method for quantifying carbon in carbides according to [5] above, wherein a quantity of the metal members used in the combustion process is such that a degree of separation calculated in the quantification process from a peak derived from contaminant carbon and a peak derived from carbon in the carbides is 1.4 or more.
[7] The method for quantifying carbon in carbides according to any one of [1] to [6] above, wherein a temperature at which the carbides and the composite member are heated in the combustion process is 700 °C or more and 1200 °C or less.
[8] The method for quantifying carbon in carbides according to any one of [1] to [7] above, wherein melting points of the metal members are 200 °C or more and 3000 °C or less, and
a difference in the melting points between the metal members is 500 °C or more and 2400 °C or less.

[9] The method for quantifying carbon in carbides according to [8] above, wherein each of the metal members includes, as the main component thereof, a metal element selected from the group consisting of Sn, Ti, Ag, Fe, Mo, and Cu.

[10] The method for quantifying carbon in carbides according to [9] above, wherein the composite member is composed of a metal member whose main component is Sn and another metal member whose main component is Fe.

[11] The method for quantifying carbon in carbides according to any one of [1] to [10] above, wherein the carbides are carbides in steel.

(Advantageous Effect)

According to the present disclosure, quantifying carbon in carbides is possible by a highly precise and simple method. Further, quantifying carbon in fine carbides is also possible.

BRIEF DESCRIPTION OF THE DRAWINGS

In the accompanying drawings:

FIG. 1A and FIG. 1B illustrate examples of infrared absorption spectra obtained in experimental examples, where FIG. 1A illustrates Comparative Example 1 and Comparative Example 6 and FIG. 1B illustrates Example 1 and Example 6;
FIG. 2A and FIG. 2B illustrate correlation between amount of carbon in carbides and peak intensity, where FIG. 2A illustrates Comparative Examples 1 to 6 and FIG. 2B illustrates Examples 1 to 6;
FIG. 3 is a diagram illustrating correlation between heating temperature in a combustion process and detection time and degree of separation of a peak top derived from carbon in carbides;
FIG. 4 is a diagram illustrating correlation between detection time of the peak top and degree of separation and amount of Fe sheet material used;
FIG. 5 is a diagram illustrating detection time of the peak top and degree of separation for Examples 15 to 18; and
FIG. 6 illustrates infrared absorption spectra obtained for Example 19 and Example 20.

DETAILED DESCRIPTION

Embodiments of the present disclosure are described below. The method for quantifying carbon in carbides includes a combustion process in which the carbides are heated together with a composite member in the presence of oxygen to oxidize the carbon in the carbides to carbon monoxide and/or carbon dioxide, and a quantification process of quantifying the carbon monoxide and/or the carbon dioxide. Here, the composite member includes two or more metal members that have main components that are different from each other.

(Carbide sample preparation)

Carbides to be analyzed are not particularly limited. Carbides to be analyzed may be, for example, carbides in steel. Carbides in steel include $TiC$, $CrC$, $Cr_3C_2$, $Cr_{23}C_6$, $NbC$, $Fe_3C$, $VC$, and the like. As carbides to be analyzed other than carbides in steel, examples include sodium carbonate and calcium carbonate.

When quantifying carbon in carbides in steel, the carbides is first extracted. Methods of extracting carbides are not particularly limited, and any method by which carbides may be extracted may be used, such as electrolytic extraction, for example.

In electrolytic extraction, carbides are dispersed in a solvent after electrolytic extraction to form a dispersion liquid, and the carbides extracted. Here, solvents are not limited, but a solvent that is relatively easy to obtain and of high purity is preferred, such as pure water, methanol, or ethanol.

Carbides dispersed in the dispersion liquid may be collected using a filter. As a filter, a filter that does not contain carbon is preferred to reduce the impact of contaminant carbon. As such a filter, an inorganic filter such as a glass filter, an alumina filter, a silver membrane filter, or the like is preferred.

Carbides dispersed in the dispersion liquid may be collected by dropping the dispersion liquid on a metal member of the composite member to be heated together in the combustion process (for example, a metal member of sheet metal worked into a container shape) and evaporating the solvent in the dispersion liquid. Heating may be used as a method to evaporate the solvent in the dispersion liquid. Too high a heating temperature may evaporate the carbides to be analyzed, and therefore the heating temperature is preferably 250 °C or less. The heating temperature is more preferably 200 °C or less. The heating temperature is even more preferably 150 °C or less. The heating temperature is even more preferably 100 °C or less. The heating temperature is even more preferably 90 °C or less. The heating temperature is even more preferably 70 °C or less. The heating temperature is even more preferably 50 °C or less. When the solvent is evaporated at room temperature, a preferred method is to evaporate the solvent in a desiccator lined with a hygroscopic agent that has not been coated with petroleum jelly or the like, in order to avoid contamination by carbon from the atmosphere.

(Combustion process)

First, the carbides are heated together with the composite member in the presence of oxygen to oxidize the carbon in the carbides to carbon monoxide and/or carbon dioxide (combustion process). The composite member heated together with the carbides includes two or more metal members that have main components that are different from each other. Accordingly, carbon in the carbides may be quantified by a highly precise and simple method by separating a peak derived from contaminant carbon from a peak derived from the carbon in the carbides. Each of the metal members is a member made of metal. Each of the metal members may be composed of a single metal. Further, each of the metal members may be composed of an alloy or an intermetallic compound. In the case of a single metal, the main component refers to a metal element of the single metal, and in the case of an alloy or intermetallic compound, the main component refers to a metal element that is most abundant among metal elements of the alloy or intermetallic compound. Concentration of the metal element of the main component in the metal member is more than 50 mass%.

The inventor focused on melting points of the metal members and conducted further studies. As a result, the inventor found that when the melting points of the metal members are 200 °C or more and 3000 °C or less and a difference in melting points between the metal members is 500 °C or more and 2400 °C or less, the peak derived from contaminant carbon and the peak derived from carbon in the carbides may be better separated and carbon in the carbides may be quantified more precisely.

Each of the metal members preferably includes, as the main component thereof, a metal element selected from the group consisting of Sn, Ti, Ag, Fe, Mo, and Cu. The composite member preferably includes a metal member whose main component is Ag and a metal member whose main component is Fe, or a metal member whose main component is Sn and a metal member whose main component is Mo, or a metal member whose main component is Sn and a metal member whose main component is Cu, or a metal member whose main component is Sn and a metal member whose main component is Ti, or a metal member whose main component is Sn and a metal member whose main component is Fe. For example, when the member heated together with the carbides are a single metal member and the metal of the metal member is Sn, the Sn melts immediately after heating in the combustion process, making it difficult to separate a peak derived from contaminant carbon from a peak derived from carbon in the carbides. Further, when the member heated together with the carbides is a single metal member and the metal of the metal member is Fe, the Fe does not melt in the combustion process and combustion of the carbides does not proceed. In contrast, when the member heated together with the carbides is a composite member including a metal member composed of Sn and a metal member composed of Fe, the melting point of Fe is lowered by Sn, and after contaminant carbon is removed, Fe melts and carbide combustion occurs, making it easier to separate a peak derived from contaminant carbon from a peak derived from carbon in the carbides. From the viewpoint of availability, workability, and purity, the composite member more preferably includes a metal member whose main component is Sn and a metal member whose main component is Fe. Each metal member is preferably composed of a single pure metal in order to more easily predict and control peak position of a peak derived from carbon of the carbides, and to reduce carbon content, as described below. Further, each metal member may contain inevitable impurity and other elements, and may contain other alloys, intermetallic compounds, and the like, as long as such content is in a range that does not interfere with the effects described herein.

The carbon in the metal member is oxidized simultaneously with the oxidation reaction of carbon in the carbides. Therefore, when measuring an amount of carbon in the carbides, a value measured is a sum of actual carbon content and an amount of carbon in the metal member. Therefore, considering an effect on quantification of carbon in carbides, the carbon content of the metal member is preferably 200 ppm or less. The carbon content of the metal member is more preferably 100 ppm or less. The carbon content of the metal member is even more preferably 10 ppm or less. The carbon content of the metal member is even more preferably 6.6 ppm or less. The carbon content of the metal member is most preferably 2 ppm or less. When the carbon content of the metal member is 200 ppm or less, then even when the carbon content varies between production lots or due to segregation or the like, the amount of carbon in the carbides may be analyzed precisely. Each of the two or more metal members of the composite member preferably meets the above requirements.

Each of the two or more metal members is preferably a sheet material having a thickness of 0.10 mm or less. When the thickness of the sheet material is 0.10 mm or less, it is easier to work into a container shape, easier to collect the dispersion liquid containing carbides on the sheet material, and easier to combust. The thickness of the sheet material is more preferably 0.05 mm or less. The thickness of the sheet material is preferably 0.001 mm or more. The thickness of the sheet material is more preferably 0.005 mm or more. When the thickness of the sheet material is 0.001 mm or more, there is no risk of the sheet material tearing or the like, and working is easier due to moderate rigidity. When all of the two or more metal members of the composite member are made of sheet material, each sheet material preferably meets the above requirements. Further, from the viewpoint of workability and ease of prediction and control of positions of a peak derived from carbon of the carbides, each of the metal members is preferably a pure metal composed of a single metal.

The carbides are preferably heated while wrapped in the sheet material. The presence of the metal member around the carbides uniformly suppresses oxidation of the carbides and improves analysis precision. First wrapping the carbides with a metal member that has a relatively low melting point and then further wrapping with a metal member that has a relatively high melting point is preferred. Specifically, when a composite member composed of a metal member whose main component is Sn and a metal member whose main component is Fe is used as the composite member, it is preferable to first wrap the carbides with the metal member whose main component is Sn and then wrap the metal member whose main component is Sn with the metal member whose main component is Fe. This allows for good separation of a peak derived from contaminant carbon from a peak derived from carbon in the carbides. Further, as long as the effect is obtainable, another member may be included in addition to the metal members of the composite member, and such cases are also included in the scope of the present disclosure.

The heating temperature of the combustion process is not limited. The heating temperature of the combustion process is preferably 700 °C or more. The heating temperature of the combustion process is preferably 1200 °C or less. When the heating temperature exceeds 1200 °C, melting of the composite member and combustion of contaminant carbon may occur almost simultaneously, making it difficult to separate a peak derived from contaminant carbon from a peak derived from carbon in the carbides to be analyzed. The heating temperature is preferably 1100 °C or less. The heating temperature is more preferably 1000 °C or less. On the other hand, when the heating temperature is less than 700 °C, melting of the composite member may not occur stably, that is, combustion of the carbides may not occur stably. The heating temperature is preferably 700 °C or more. The heating temperature is more preferably 800 °C or more. The heating temperature is even more preferably 900 °C or more.

(Quantification process)

The analysis method for quantifying carbon monoxide and/or carbon dioxide is not particularly limited, and may be any analysis method able to quantify carbon monoxide and/or carbon dioxide generated in the combustion process. The analysis method is preferably quantification by an infrared absorption method.

When infrared absorption is used as the analysis method, it is preferable to use in the quantification process a quantity of the metal members such that a degree of separation calculated from a peak derived from contaminant carbon and a peak derived from carbon in the carbides is 1.4 or more. The degree of separation is more preferably 1.7 or more. When the degree of separation is less than 1.4, the metal members melt quickly and a peak derived from carbon in the carbides approaches a position of a peak derived from contaminant carbon, which reduces the separation precision of the two peaks. Here, the degree of separation is a value calculated from the following formula.

$$\text{Degree of separation R} = 1.18 \times (tR2 - tR1) / (W1 + W2)$$

Here, tR1 and tR2 are times when a peak top of contaminant carbon and carbon in carbides is detected, respectively, and W1 and W2 are the full widths at half maximum of the peaks of the contaminant carbon and the carbon in the carbides, respectively.

EXAMPLES

Experimental examples of the present disclosure are described below, but the present disclosure is not limited to the examples described.

(Comparative Examples 1 to 6)

First, a single metal member was prepared as a member to be combusted together with carbides in the combustion process. Here, Sn capsules (produced by Ludi Swiss AG, thickness: 0.01 mm, size: 5 mm in diameter $\times$ 9 mm in height, mass: 27 mg, purity: 99.999 mass%) made of Sn sheet material having a C content of 1.6 ppm were prepared. Further, sodium carbonate having a purity of 99.0 mass% or more, produced by Tokyo Chemical Industry Co., Ltd., was prepared as the carbides. The sodium carbonate was then added to pure water and stirred to produce a dispersion liquid of sodium carbonate as the carbides. The prepared dispersion liquid was aliquoted into the Sn capsules with carbon amounts of 0 $\mu$g (Comparative Example 1), 0.1 $\mu$g (Comparative Example 2), 0.3 $\mu$g (Comparative Example 3), 0.5 $\mu$g

(Comparative Example 4), 1.0 μg (Comparative Example 5), and 1.5 μg (Comparative Example 6), and the pure water was evaporated by heating at 90 °C for 2 h, to extract the carbides in the Sn capsules. For Comparative Example 1, about 0.1 ml of pure water was placed in the Sn capsule and the pure water was evaporated. The carbides were then wrapped by folding the Sn capsule (wrapped in Sn sheet material) and placed in a ceramic crucible. The Sn capsule was then heated to 800 °C in the presence of $O_2$, and peak positions and intensity of generated carbon monoxide and/or carbon dioxide were measured by infrared absorption.

(Examples 1 to 6)

Quantification of carbon in carbides was performed in the same manner as for Comparative Examples 1 to 6. However, a composite member was used as the member combusted along with the carbides. As the composite member, a composite member composed of two metal members whose main components are different from each other was used, the two metal members being a Sn capsule (produced by Ludi Swiss AG, thickness: 0.01 mm, size: 5 mm in diameter × 9 mm in height, mass: 27 mg, melting point: 232 °C, purity: 99.999 mass%) made of Sn sheet material having a C content of 1.6 ppm and Fe sheet material having a C content of 2 ppm (produced by Toho Zinc Co., Ltd., Mairon®-UHP (Mairon is a registered trademark in Japan, other countries, or both), thickness: 0.005 mm, 10 mm × 10 mm, mass: 4 mg, melting point: 1538 °C, purity: 99.999 mass%). The melting point difference between the metal members of the composite member was 1306 °C. Further, the dispersion liquid of sodium carbonate as the carbides was aliquoted into the Sn capsules with carbon amounts of 0 μg (Example 1), 0.1 μg (Example 2), 0.3 μg (Example 3), 0.5 μg (Example 4), 1.0 μg (Example 5), and 1.5 μg (Example 6), and the pure water was evaporated by heating at 90 °C for 2 h to extract the carbides in the Sn capsules. The carbides were then wrapped by folding the Sn capsule, and subsequently the Sn capsule was wrapped with the Fe sheet material, and placed in a ceramic crucible. All other conditions were the same as for Comparative Examples 1 to 6.

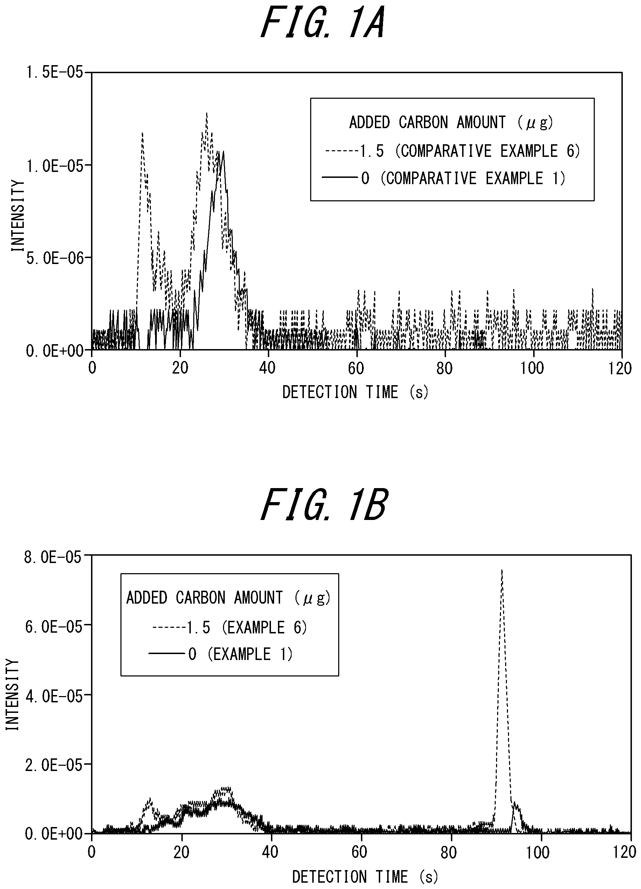

FIG. 1A and FIG. 1B illustrate examples of infrared absorption spectra obtained, where FIG. 1A illustrates Comparative Example 1 and Comparative Example 6 and FIG. 1B illustrates Example 1 and Example 6. For the spectrum of Comparative Example 1 in FIG. 1A, the detection time of the peak top was around 30 s. This is the peak derived from contaminant carbon. On the other hand, for the spectrum of Comparative Example 6 in FIG. 1A, peaks were detected twice, with the detection time of the first peak top around 10 s and the detection time of the second peak top around 25 s. The first peak is considered to be a peak derived from carbon in the carbides and the second peak is considered to be derived from contaminant carbon. Peak intensity derived from carbon in the carbides is less than or equivalent to peak intensity derived from contaminant carbon, indicating strong influence by contaminant carbon.

In contrast, for the spectrum of Example 6 in FIG. 1B, two peaks were also detected, with the detection time of the first peak top around 30 s and the detection time of the second peak top around 90 s. The first peak is considered to be a peak derived from contaminant carbon, and the second peak is considered to be a peak derived from carbon in the carbides. The results demonstrate that when a composite member composed of a Sn capsule and Fe sheet material is used as a metal member that combusts together with the carbides, it is possible to clearly separate the peak derived from contaminant carbon from the peak derived from carbon in the carbides. The degrees of separation for Examples 2 to 6 were 4.3 (Example 2), 4.5 (Example 3), 3.6 (Example 4), 2.7 (Example 5), and 3.2 (Example 6), respectively. However, as is clear from the spectrum of Example 1, even when the Sn capsule and Fe sheet material containing pure water without carbides were heated, a peak was detected at around a detection time of 90 s. This is considered to be derived from carbon contained in the Fe sheet material. The effect of carbon in Fe sheet material may be reduced by creating and correcting a calibration curve.

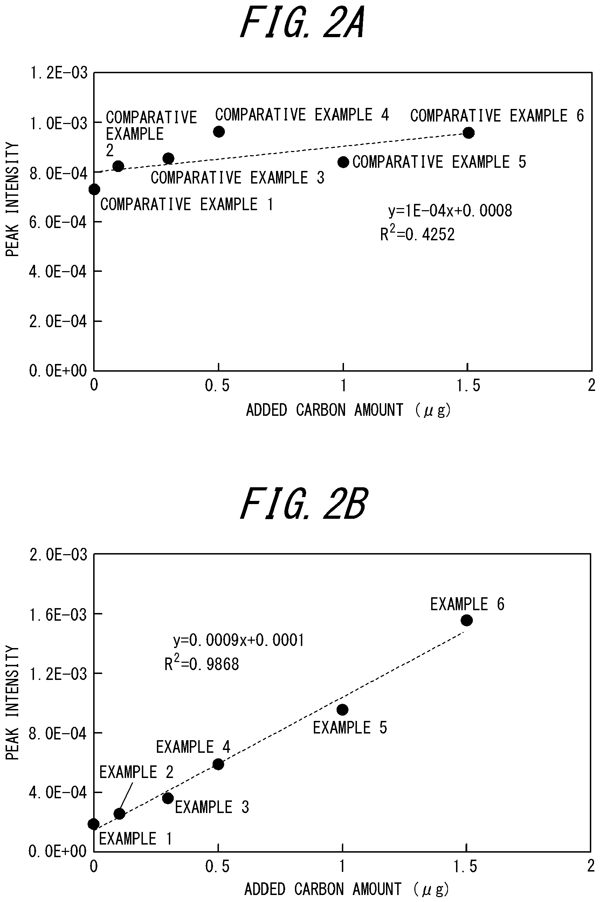

FIG. 2A and FIG. 2B illustrate correlation between amount of carbon in the carbides and peak intensity, where FIG. 2A illustrates Comparative Examples 1 to 6 and FIG. 2B illustrates Examples 1 to 6. From FIG. 2A, it can be seen that for Comparative Examples 1 to 6, where the member combusted together with the carbides is a single metal member composed of only a Sn capsule, fitting with a straight line results in a small slope, and the correlation coefficient R is low at 0.4252, indicating that linearity is not favorable. In contrast, from FIG. 2B it can be seen that for Examples 1 to 6, where the member combusted together with the carbides is a composite member composed of two metal members having different main components, one a Sn capsule and the other an Fe sheet material, fitting with a straight line results in a large slope, and the correlation coefficient R is high at 0.9868, indicating that linearity is also favorable.

When the dispersion liquid in which the carbides was dispersed was dropped directly onto the Fe sheet material and dried, and the carbide wrapped in the Fe sheet material then heated, the detection time of the peak top was only around 10 s. This is considered to be because the Fe sheet material did not melt in the combustion process and the carbides did not combust.

Further, the sample to which no carbides were added was used as a blank, the blank sample was measured three times, the standard deviation was calculated from the values obtained from the blank samples, and the calculated standard deviation was multiplied by 10 to obtain a lower limit of quantification of carbon amount. As a result, the lower limit of quantification of carbon amount was 6.5 μg when only a Sn capsule (single metal member) was used as a member, and 0.11 μg when a composite member composed of two metal members whose main components differ from each other, one a Sn capsule and one an Fe sheet material, was used. From these results, it was confirmed that the use of a composite member composed of two metal members, a Sn capsule and an Fe sheet material, as a member combusted together with carbides, improves analysis precision.

(Examples 7 to 10)

Quantification of carbon in carbides was performed in the same manner as for Examples 1 to 6. However, a dispersion liquid of sodium carbonate was aliquoted to obtain carbon amounts of 20 μg. Further, the heating temperatures in the combustion process were 700 °C (Example 7), 800 °C (Example 8), 900 °C (Example 9), and 1000 °C (Example 10), respectively. All other conditions were the same as for Examples 1 to 6.

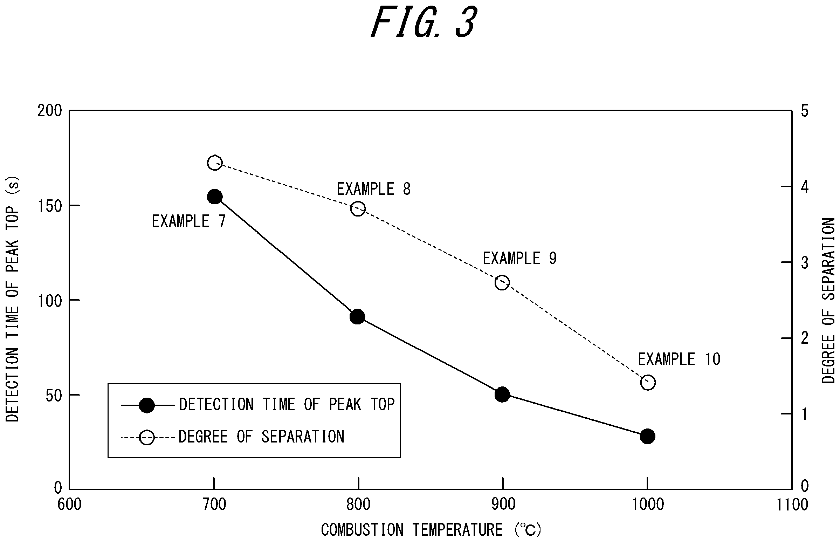

FIG. 3 illustrates correlation between heating temperature in the combustion process and detection time and degree of separation of a peak top derived from carbon in the carbides. FIG. 3 clearly illustrates that the detection time of the peak top decreases with increasing heating temperature. The results indicate that increasing the heating temperature makes it possible to shorten the detection time of the peak top, that is, the measurement time. On the other hand, although the degree of separation decreases with increasing heating temperature, values of 1.4 or more are indicated, indicating that the separation precision between the peak derived from carbon in the carbides and the peak derived from contaminant carbon is sufficient. From the above, it can be seen that efficiency of analysis may be improved by increasing the heating temperature, while securing analysis precision.

(Examples 11 to 14, Comparative Example 7)

Quantification of carbon in carbides was performed in the same manner as for Example 7. However, the amount of Fe sheet material used was 0 mg (Comparative Example 7), 8 mg (Example 11), 16 mg (Example 12), 32 mg (Example 13), and 64 mg (Example 14), respectively. All other conditions were the same as for Example 7.

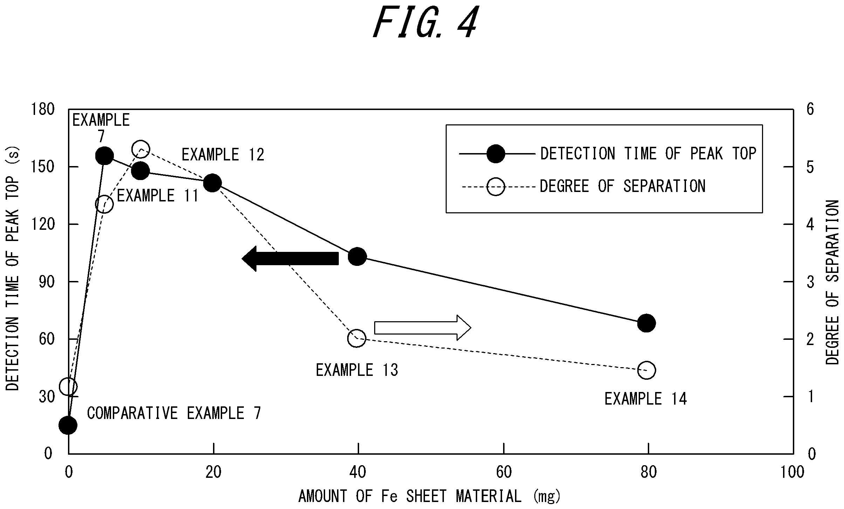

FIG. 4 illustrates correlation between detection time of a peak top derived from carbon in the carbides and degree of separation and amount of Fe sheet material used. It can be seen that the detection time of the peak top decreases as the amount of Fe sheet material used increases. This result indicates that increasing the amount of Fe sheet material used is able to shorten the detection time of the peak top, that is, the measurement time. On the other hand, although the degree of separation tends to decrease as the amount of Fe sheet material used increases, values of 1.4 or more are indicated for the separation degree, indicating that the separation precision between the peak derived from carbon in the carbides and the peak derived from contaminant carbon is sufficient. From the above, it can be seen that efficiency of analysis may be improved by increasing the amount of Fe sheet material used, while securing analysis precision.

(Examples 15 to 18)

Quantification of carbon in carbides was performed in the same manner as for Example 9. However, instead of the Sn capsule of Example 9, an Ag capsule (produced by Ludi Swiss AG, thickness: 0.01 mm, size: 5 mm in diameter × 9 mm in height, mass: 42 mg, melting point: 962 °C, purity: 99.99 mass%) made of Ag sheet material having a C content of 56 ppm was used for Example 15. Melting point difference between the metal members of the composite member was 576 °C. Further, instead of the Fe sheet material of Example 9, a Mo sheet material (thickness: 0.01 mm, 10 mm × 10 mm, mass: 10 mg, melting point: 2623 °C, purity: 99.99 mass%) having a C content of 16 ppm was used for Example 16, where melting point difference between the metal members of the composite member was 2391 °C. A Cu sheet material (thickness: 0.01 mm, 10 mm × 10 mm, mass: 9 mg, melting point: 1085 °C, purity: 99.99 mass%) having a C content of 68 ppm was used for Example 17, where melting point difference between the metal members of the composite member was 853 °C. A Ti sheet material (thickness: 0.01 mm, 10 mm × 10 mm, mass: 5 mg, melting point: 1668 °C, purity: 99.9 mass%) having a C content of 159 ppm was used for Example 18, where melting point difference between the metal members of the composite member was 1436 °C. All other conditions were the same as for Example 9.

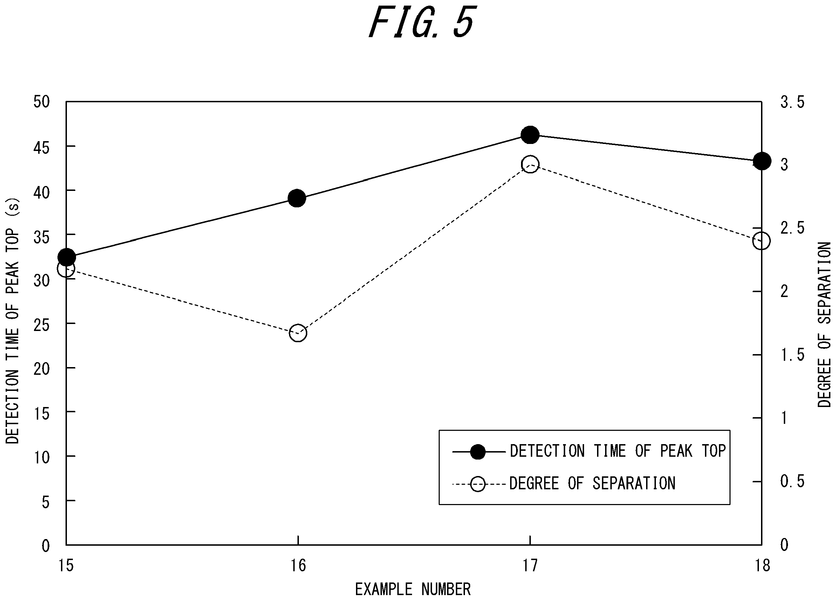

FIG. 5 illustrates detection time of the peak top and degree of separation for Examples 15 to 18. The degree of separation for Examples 15 to 18 was 1.5 or more, indicating that separation precision between peaks derived from carbon in the carbides and peaks derived from contaminant carbon was sufficient. Further, the detection time of the peak top ranged from 32 s to 48 s, indicating that the efficiency of the analysis was sufficient.

(Examples 19 and 20) Quantification of carbon in carbides was performed in the same manner as for Examples 1 to 6. However, for Example 19, instead of a dispersion liquid in which sodium carbonate was dispersed as the carbides, a dispersion liquid in which TiC (produced by Kojundo Chemical Lab. Co., Ltd., purity 99 mass% or more) was dispersed was used to simulate carbides in steel, and the amount of carbon was 2.0 μg. Further, for Example 20, a dispersion

liquid in which $Cr_3C_2$ (produced by Strem Chemicals Inc., purity 97.5 mass% or more) was dispersed was used, and the amount of carbon was 2.0 μg. All other conditions were the same as for Examples 1 to 6.

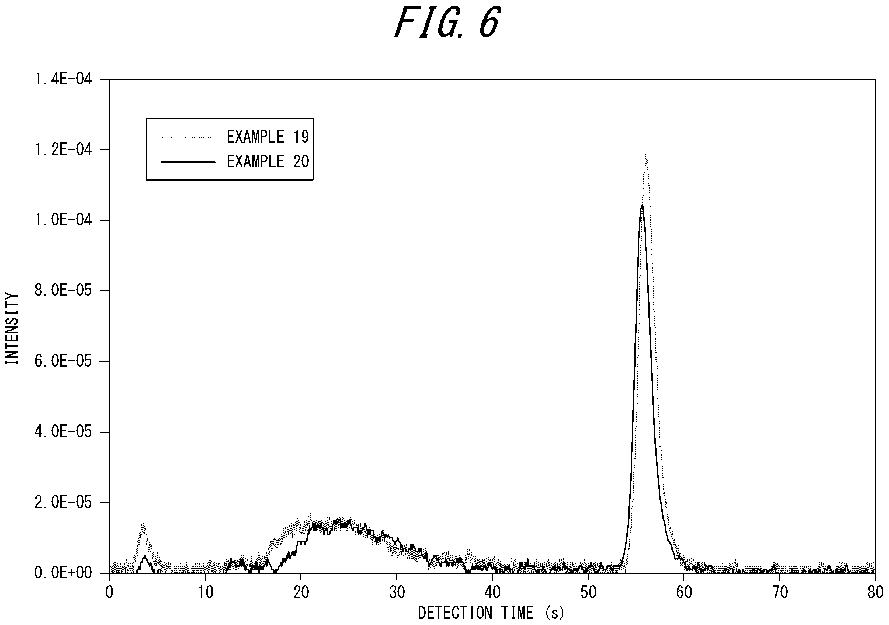

FIG. 6 illustrates infrared absorption spectra obtained for Example 19 and Example 20. As in Example 6, a peak derived from contaminant carbon appeared at around 24 s, and a peak derived from carbon in the carbides appeared at around 55 s, indicating that the effect of the present disclosure is obtainable when carbides commonly contained in steel are the target of analysis.

INDUSTRIAL APPLICABILITY

The present disclosure makes quantifying carbon in carbides by a highly precise and simple method possible, and is therefore useful in the iron and steelmaking industry.

**Claims**

1. A method for quantifying carbon in carbides, comprising: a combustion process in which the carbides are heated together with a composite member in the presence of oxygen to oxidize the carbon in the carbides to carbon monoxide and/or carbon dioxide; and a quantification process of quantifying the carbon monoxide and/or the carbon dioxide, wherein
   the composite member includes two or more metal members that have main components that are different from each other.

2. The method for quantifying carbon in carbides according to claim 1, wherein carbon content of each of the two or more metal members is 200 ppm or less.

3. The method for quantifying carbon in carbides according to claim 1 or 2, wherein each of the two or more metal members is composed of a sheet material having a thickness of 0.10 mm or less.

4. The method for quantifying carbon in carbides according to claim 3, wherein the carbides are heated while wrapped in the sheet material in the combustion process.

5. The method for quantifying carbon in carbides according to any one of claims 1 to 4, wherein an analysis method in the quantification process is an infrared absorption method.

6. The method for quantifying carbon in carbides according to claim 5, wherein a quantity of the metal members used in the combustion process is such that a degree of separation calculated in the quantification process from a peak derived from contaminant carbon and a peak derived from carbon in the carbides is 1.4 or more.

7. The method for quantifying carbon in carbides according to any one of claims 1 to 6, wherein a temperature at which the carbides and the composite member are heated in the combustion process is 700 °C or more and 1200 °C or less.

8. The method for quantifying carbon in carbides according to any one of claims 1 to 7, wherein melting points of the metal members are 200 °C or more and 3000 °C or less, and
   a difference in the melting points between the metal members is 500 °C or more and 2400 °C or less.

9. The method for quantifying carbon in carbides according to claim 8, wherein each of the metal members includes, as the main component thereof, a metal element selected from the group consisting of Sn, Ti, Ag, Fe, Mo, and Cu.

10. The method for quantifying carbon in carbides according to claim 9, wherein the composite member is composed of a metal member whose main component is Sn and another metal member whose main component is Fe.

11. The method for quantifying carbon in carbides according to any one of claims 1 to 10, wherein the carbides are carbides in steel.

## FIG. 1A

## FIG. 1B

## FIG. 2A

## FIG. 2B

## FIG. 3

# FIG. 4

# FIG. 5

EP 4 450 967 A1

# FIG. 6

## INTERNATIONAL SEARCH REPORT

| International application No. |
| --- |
| **PCT/JP2022/046727** |

### A. CLASSIFICATION OF SUBJECT MATTER

*G01N 31/00*(2006.01)i; *G01N 31/12*(2006.01)i; *G01N 33/2022*(2019.01)i; *G01N 21/3563*(2014.01)i
FI: G01N31/12 B; G01N21/3563; G01N31/00 E; G01N33/2022

According to International Patent Classification (IPC) or to both national classification and IPC

### B. FIELDS SEARCHED

Minimum documentation searched (classification system followed by classification symbols)

G01N31/00; G01N31/12; G01N33/2022; G01N21/3563

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| Y | JP 2-136743 A (NIPPON STEEL CORP.) 25 May 1990 (1990-05-25) claims (1)-(5), (Industrial field of application) | 1-3, 5-9, 11 |
| A | | 4, 10 |
| Y | JP 63-9861 A (NIPPON STEEL CORP.) 16 January 1988 (1988-01-16) claims, p. 2, lower right column, lines 1-14, p. 3, lower right column, line 19 to p. 4, upper left column, line 11, p. 4, lower right column, line 20, p. 5, upper left column, line 4, example -3 | 1-3, 5-9, 11 |
| Y | JP 2012-37244 A (SUMITOMO METAL MINING CO., LTD.) 23 February 2012 (2012-02-23) paragraphs [0006], [0007] | 1-3, 5-9, 11 |
| A | JP 6-87057 B2 (NIPPON STEEL CORP.) 02 November 1994 (1994-11-02) p. 2, left column, lines 15-32 | 1-11 |
| A | JP 10-260175 A (HORIBA, LTD.) 29 September 1998 (1998-09-29) claim 1, paragraphs [0008], [0014] | 1-11 |

☑ Further documents are listed in the continuation of Box C.  ☑ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
| --- | --- |
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **07 February 2023** | **21 February 2023** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**

International application No.

**PCT/JP2022/046727**

| C. | DOCUMENTS CONSIDERED TO BE RELEVANT | |
|---|---|---|
| Category* | Citation of document, with indication, where appropriate, of the relevant passages | Relevant to claim No. |
| A | JP 9-166590 A (NKK CORP.) 24 June 1997 (1997-06-24)<br>paragraphs [0003], [0004], [0015], [0026] | 1-11 |
| A | JP 61-280564 A (SUMITOMO METAL INDUSTRIES, LTD.) 11 December 1986<br>(1986-12-11)<br>claims | 1-11 |
| A | JP 2008-224462 A (TAIHEIYO CEMENT CORP.) 25 September 2008 (2008-09-25)<br>abstract | 1-11 |
| A | JP 6-317576 A (NIPPON STEEL CORP.) 15 November 1994 (1994-11-15)<br>paragraph [0009] | 1-11 |
| A | JP 5-346426 A (NIPPON STEEL CORP.) 27 December 1993 (1993-12-27)<br>abstract | 1-11 |
| A | US 2020/0150025 A1 (EXXONMOBIL RESEARCH AND ENGINEERING CO.) 14 May<br>2020 (2020-05-14)<br>abstract | 1-11 |
| A | JP 11-44687 A (KAWASAKI STEEL CORP.) 16 February 1999 (1999-02-16)<br>entire text, all drawings | 1-11 |
| A | JP 2006-250098 A (HITACHI, LTD.) 21 September 2006 (2006-09-21)<br>paragraph [0002] | 1-11 |
| A | JP 2019-39710 A (NIPPON STEEL & SUMITOMO METAL CORP.) 14 March 2019<br>(2019-03-14)<br>entire text, all drawings | 1-11 |

Form PCT/ISA/210 (second sheet) (January 2015)

## INTERNATIONAL SEARCH REPORT
### Information on patent family members

International application No.

**PCT/JP2022/046727**

| Patent document cited in search report | | | Publication date (day/month/year) | Patent family member(s) | | | Publication date (day/month/year) |
|---|---|---|---|---|---|---|---|
| JP | 2-136743 | A | 25 May 1990 | (Family: none) | | | |
| JP | 63-9861 | A | 16 January 1988 | (Family: none) | | | |
| JP | 2012-37244 | A | 23 February 2012 | (Family: none) | | | |
| JP | 6-87057 | B2 | 02 November 1994 | JP | 3-37566 | A | |
| JP | 10-260175 | A | 29 September 1998 | (Family: none) | | | |
| JP | 9-166590 | A | 24 June 1997 | (Family: none) | | | |
| JP | 61-280564 | A | 11 December 1986 | (Family: none) | | | |
| JP | 2008-224462 | A | 25 September 2008 | (Family: none) | | | |
| JP | 6-317576 | A | 15 November 1994 | (Family: none) | | | |
| JP | 5-346426 | A | 27 December 1993 | (Family: none) | | | |
| US | 2020/0150025 | A1 | 14 May 2020 | (Family: none) | | | |
| JP | 11-44687 | A | 16 February 1999 | (Family: none) | | | |
| JP | 2006-250098 | A | 21 September 2006 | (Family: none) | | | |
| JP | 2019-39710 | A | 14 March 2019 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H1144687 A

- JP 2004257920 A